# EUROPEAN PATENT APPLICATION

(11) **EP 1 491 219 A2**
(43) Date of publication of application: **29.12.2004**
(21) Application number: 04014571.6
(22) Date of filing: 22.06.2004
(51) Int. Cl.: A61L 15/58

(54) **Pressure-sensitive adhesive sheet for application to skin and first-aid plaster**

(30) Priority: 24.06.2003 JP 2003179230
(71) Applicant: NITTO DENKO CORPORATION, Osaka (JP)
(72) Inventor: Kuniya, Masayoshi, Ibaraki-shi Osaka (JP); Suzuki, Seishi, Ibaraki-shi Osaka (JP); Sasaki, Yasuyuki, Ibaraki-shi Osaka (JP)
(74) Representative: Grünecker, Kinkeldey, Stockmair & Schwanhäusser Anwaltssozietät

(57) **Abstract**

A pressure-sensitive adhesive sheet for application to the skin which employs a substrate film made of a chlorine-free material is provided as a substitute for the substrate film made of a flexible vinyl chloride resin. A pressure-sensitive adhesive sheet for application to the skin, which comprises (1) a substrate film comprising a resin composition comprising from 20 to 80% by weight of an ethylene/vinyl acetate copolymer having a weight-average molecular weight higher than 1×10⁵ and not higher than 1×10⁶, a melt flow rate of 1.0 g/10 min or lower, and a vinyl acetate content higher than 28% by weight and not higher than 35% by weight and from 80 to 20% by weight of at least one polyolefin resin selected from the group consisting of polyethylene, polypropylene, ethylene/propylene copolymers, and mixtures thereof and having a melt flow rate in the range of from 0.3 to 4.0 g/10 min and, (2) a pressure-sensitive adhesive layer for application to the skin.

## Description

### FIELD OF THE INVENTION

The present invention relates to a pressure-sensitive adhesive sheet for application to the skin, which comprises a substrate film comprising a resin composition comprising an ethylene/vinyl acetate copolymer and a polyolefin resin and a pressure-sensitive adhesive layer formed on the substrate film, as well as to uses of the pressure-sensitive adhesive sheet. More particularly, the present invention relates to a pressure-sensitive adhesive sheet for application to the skin which has all of the important properties required for pressure-sensitive adhesive sheets for application to the skin, e.g., the following properties: to be flexible and well conform to the skin to give a good feeling during wear (wear feeling); to have practically sufficient strength despite the flexibility; to have excellent surface slip properties so as not to peel off upon friction with clothes, etc. during wear; and to be prevented from considerably curling (bending) due to a stress generated when the pressure-sensitive adhesive sheet having a release paper is separated from the release paper. The present invention further relates to uses of the pressure-sensitive adhesive sheet, e.g., use as a first-aid plaster.

### BACKGROUND OF THE INVENTION

Pressure-sensitive adhesive sheets for application to the skin which comprise a flexible substrate film and a pressure-sensitive adhesive layer formed thereon have hitherto been used as dressings, surgical tapes, etc. Besides being used in such applications, the pressure-sensitive adhesive sheets are widely used in general homes as a first-aid plaster produced by disposing a liquid-absorbing pad, e.g., a gauze, in a central area of the surface of the pressure-sensitive adhesive layer. Furthermore, pressure-sensitive adhesive drug sheets for application to the skin which comprise a substrate film and formed thereon a layer of a pressure-sensitive adhesive containing any of various drugs are being used in various medical applications.

As the substrate films for such pressure-sensitive adhesive sheets for application to the skin, films made of a so-called flexible vinyl chloride resin have hitherto been widely used. This is because these substrate films not only are flexible and well conform to the skin to give a good wear feeling but also have excellent processability and printability.

However, since flexible vinyl chloride resins are produced by incorporating a large amount of a plasticizer such as, e.g., dioctyl phthalate into poly(vinyl chloride) in order to impart flexibility thereto, there have been the following and other problems. Such a plasticizer migrates to the pressure-sensitive adhesive layer to reduce the cohesive force of the pressure-sensitive adhesive layer, and this results in a decrease in adhesive force and causes the so-called adhesive remaining on the skin surface which has been covered with the adhesive sheet.

Furthermore, it is known that flexible vinyl chloride resins are highly temperature-sensitive and change in properties with ambient temperature. When a flexible vinyl chloride resin is used as a substrate film for a pressure-sensitive adhesive sheet for application to the skin, the substrate film becomes rigid, i.e., becomes less flexible, upon exposure to low temperatures in winter or during kitchen work. As a result, there are cases where the pressure-sensitive adhesive sheet gives an impaired wear feeling or has reduced conformability to the skin to show peeling or lifting from the skin. In the case where the substrate film of the pressure-sensitive adhesive sheet applied to a finger of a hand becomes highly rigid, there is even the possibility that the substrate film might damage the skin of the face during face washing.

In addition, substitution of chlorine-free materials in various vinyl chloride resin products is proceeding recently in various fields while taking account of influences on the environment. Polyolefin resins such as polyethylene and polypropylene are regarded as important proposed substitute materials from the standpoints of profitability and safety.

Under these circumstances, also in the field of substrate films for pressure-sensitive adhesive sheets for application to the skin, substrate films made of a polyolefin resin such as polyethylene or polypropylene have been investigated as substitutes for the substrate films made of a flexible vinyl chloride resin.

For example, in producing a substrate film made of a polyolefin resin such as polyethylene or polypropylene, an attempt has been made to reduce the thickness of the substrate film for the purpose of imparting thereto the same flexibility as that of the substrate films heretofore in use which are made of a flexible vinyl chloride resin and to incorporate an elastomer ingredient for the purpose of imparting stretchability thereto. However, the substrate film thus obtained has insufficient mechanical strength. In particular, it has had a drawback that when many through-holes having a diameter of about 1 mm are formed in this pressure-sensitive adhesive sheet in order to mitigate skin moistening during the period of the wear of the pressure-sensitive adhesive sheet, then there are cases where the pressure-sensitive adhesive sheet breaks when peeled from the skin.

A substrate film for use in pressure-sensitive adhesive sheets has hence been proposed which is obtained from, as a material therefor, an ethylene/vinyl acetate copolymer having properties in certain ranges, i.e., a weight-average molecular weight (Mw), molecular-weight distribution, melt flow rate (MFR), and vinyl acetate content which are within certain ranges (see document 1). However, the ethylene/vinyl acetate copolymer used for it has a low melt viscosity during calendering and, hence, film production therefrom by calendering is apt to encounter troubles such as film sagging, breakage, etc. when the film is peeled from the rolls. In particular, it has been exceedingly difficult to produce a thin film having a thickness of 100 µm or smaller by calendering. Although increasing the film thickness is effective in avoiding such problems in some degree, this results in an increase in film production cost in vain.

Furthermore, in the case where the film obtained by calendering is wound into a roll, the film is blocked. Because of this, the feeding of the film from the roll, i.e., unwinding the roll, in producing a pressure-sensitive adhesive sheet necessitates a force and a tension is imposed on the film. Consequently, the pressure-sensitive adhesive sheet obtained using this film shrinks or curls due to the residual stress, and winding this pressure-sensitive adhesive sheet into a roll may encounter troubles such as the so-called tightening, telescoping, etc.

In addition, since the known films made of an ethylene/vinyl acetate copolymer have poor surface slip properties, they have had a drawback that when they are used as the substrate films of pressure-sensitive adhesive sheets for application to the skin or as substrate films for plasters, the adhesive sheets or plasters readily peel off upon friction with clothes, etc.

On the other hand, polyolefin resins having a moderate melt viscosity, in particular, the so-called reactor TPO resins (olefin-based thermoplastic elastomers), have hitherto been known. Films obtained from such polyolefin resins through calendering are also already known (see, for example, document 2). Such reactor TPO resins having excellent calenderability. However, when the film obtained is used as a substrate film for a pressure-sensitive adhesive sheet for application to the skin, the film is highly susceptible to the so-called curling, i.e., the film readily curls due to the stress generated upon removal of the release paper from the pressure-sensitive adhesive sheet. There have hence been problems, for example, that the pressure-sensitive adhesive sheet is difficult to apply to the skin and, in some cases, unable to be applied so as to come into close contact with the skin.
Document 1: JP-A-11-206869
Document 2: JP-A-2001-131383

### SUMMARY OF THE INVENTION

The present inventors made extensive investigations in order to overcome the problems in the above-described substitutes for those substrate films for pressure-sensitive adhesive sheets which are made of a flexible vinyl chloride resin. As a result, the following have been found. A resin composition comprising an ethylene/vinyl acetate copolymer having properties in certain ranges and a polyolefin resin stably gives a thin film through calendering, and this film, when used as a substrate film for a pressure-sensitive adhesive sheet for application to the skin, is flexible, well conforms to the skin, and hence gives an excellent wear feeling after application to the skin. In addition, the film has excellent surface slip properties and does not curl considerably. This film has hence been found to be advantageously usable as a substrate film for pressure-sensitive adhesive sheets for application to the skin which include first-aid plasters. The present invention has been achieved based on these findings.

Namely, the present invention provides a pressure-sensitive adhesive sheet for application to the skin, which comprises (1) a substrate film comprising a resin composition comprising from 20 to 80% by weight of an ethylene/vinyl acetate copolymer having a weight-average molecular weight higher than 1×10⁵ and not higher than 1×10⁶, a melt flow rate of 1.0 g/10 min or lower, and a vinyl acetate content higher than 28% by weight and not higher than 35% by weight and from 80 to 20% by weight of at least one polyolefin resin selected from the group consisting of polyethylene, polypropylene, ethylene/propylene copolymers, and mixtures thereof and having a melt flow rate in the range of from 0.3 to 4.0 g/10 min, and (2) a pressure-sensitive adhesive layer for application to the skin.

The present invention further provides a first-aid plaster which comprises the pressure-sensitive adhesive sheet for application to the skin and a liquid-absorbing pad disposed on the surface of the pressure-sensitive adhesive layer (preferably, in the central area).

The present invention furthermore provides a process for producing the pressure-sensitive adhesive sheet for application to the skin, which comprises forming the resin composition into a film by calendering and forming a pressure-sensitive adhesive layer on the substrate film thus obtained.

### BRIEF DESCRIPTION OF THE DRAWINGS

By way of example and to make the description more clear, reference is made to the accompanying drawing in which:

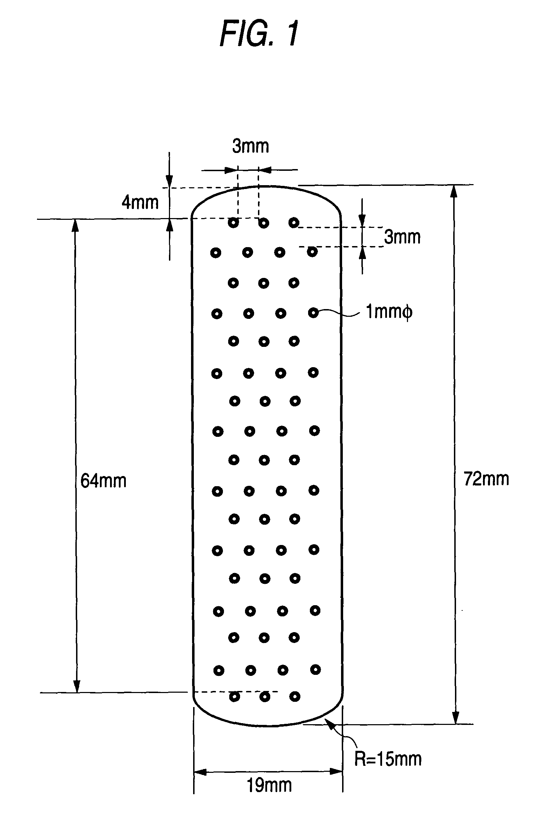

Fig. 1 illustrates a pressure-sensitive adhesive sheet which had many through-holes and was subjected to a peel strength measurement.

### DETAILED DESCRIPTION OF THE INVENTION

The pressure-sensitive adhesive sheet for application to the skin according to the present invention comprises a substrate film and, formed thereon, a pressure-sensitive adhesive layer for application to the skin. According to the present invention, the substrate film comprises a resin composition comprising from 20 to 80% by weight of an ethylene/vinyl acetate copolymer having a weight-average molecular weight (Mw) higher than 1×10⁵ and not higher than 1×10⁶, a melt flow rate (MFR) of 1.0 g/10 min or lower, and a vinyl acetate content higher than 28% by weight and not higher than 35% by weight and from 80 to 20% by weight of at least one polyolefin resin selected from the group consisting of polyethylene, polypropylene, ethylene/propylene copolymers, and mixtures thereof and having a melt flow rate in the range of from 0.3 to 4.0 g/10 min.

In the present invention, melt flow rate is measured in accordance with JIS K-6730.

In case where the ethylene/vinyl acetate copolymer to be used has a weight-average molecular weight of 1×10⁵ or lower, the film obtained may have practically insufficient mechanical strength. Especially when a thin film having a thickness of 100 µm or smaller obtained therefrom is used as a substrate film in a pressure-sensitive adhesive sheet for application to the skin, troubles such as breakage are apt to arise when the resultant pressure-sensitive adhesive sheet for application to the skin is peeled from the skin. However, even an ethylene/vinyl acetate copolymer having a weight-average molecular weight exceeding 1×10⁵ may give a film which has poor flexibility and may be unsuitable for use as a substrate film for pressure-sensitive adhesive sheets for application to the skin, when the vinyl acetate content of the ethylene/vinyl acetate copolymer is 28% by weight or lower.

On the other hand, in case where the vinyl acetate content of the ethylene/vinyl acetate copolymer exceeds 35% by weight, the film obtained is highly apt to be blocked when wound into a roll. Because of this, when the film is unwound from the roll and used as a substrate film, a tension may be imposed on the film because the film may not be smoothly released from the roll. As a result, the pressure-sensitive adhesive sheet obtained is apt to suffer shrinkage, curling, or the like due to the residual stress. Furthermore, there are cases where when such a pressure-sensitive adhesive sheet is wound into a roll, troubles such as the so-called tightening and telescoping may arise as stated above. In addition, the film obtained further has poor surface slip properties.

However, in case where the weight-average molecular weight of ethylene/vinyl acetate copolymer exceeds 1×10⁶, the copolymer gives a film having rough surfaces and an even film may not be obtained therefrom.

Furthermore, in case where the melt flow rate of the ethylene/vinyl acetate copolymer to be used exceeds 1.0 g/10 min, resin adhesion to rolls is apt to occur in calendering because such a copolymer has a low melt viscosity. In particular, the so-called roll sticking in which the film may not be taken off the final roll of a calender occurs, and troubles such as drawdown and breakage may arise when the film is taken off the calender rolls. It may be difficult to obtain a film which has smooth surfaces and has no defects. However, also in case where the melt flow rate of the ethylene/vinyl acetate copolymer to be used is too low, the copolymer may give a film having rough surfaces and an even film may not be obtained therefrom. Usually, films are printed for the purpose of, e.g., giving an attractive appearance. However, since surface roughness of films causes insufficient ink transfer in printing, etc., a film having rough surfaces is unsuitable for use as a substrate film for pressure-sensitive adhesive sheets for application to the skin. According to the present invention, it is preferred that the ethylene/vinyl acetate copolymer to be used have a melt flow rate of 0.05 g/10 min or higher.

Consequently, according to the present invention, the ethylene/vinyl acetate copolymer to be used is one which has a weight-average molecular weight (Mw) in the range of exceeding 1×10⁵ and not higher than 1×10⁶ a melt flow rate (MFR) of 1.0 g/10 min or lower, preferably in the range of from 0.05 to 0.5 g/10 min, and a vinyl acetate content higher than 28% by weight and not higher than 35% by weight. In particular, according to the present invention, the vinyl acetate content in the ethylene/vinyl acetate copolymer is preferably in the range of from 30 to 33% by weight.

However, even such an ethylene/vinyl acetate copolymer, when used alone in film formation therefrom by calendering, may give a film having poor surface slip properties. Consequently, the pressure-sensitive adhesive drug sheet for application to the skin obtained using such a film as a substrate film, after application to the skin, is apt to readily peel off the skin due to friction with clothes.

Consequently, according to the present invention, a resin composition having excellent calenderability can be obtained by using an ethylene/vinyl acetate copolymer having the properties described above in an amount of 80% by weight or smaller and using a polyolefin resin in combination therewith. In addition, by calendering such a resin composition, a substrate film suitable for use as a substrate film for pressure-sensitive adhesive sheets for application to the skin which include first-aid plasters can be obtained.

However, even a resin composition comprising the ethylene/vinyl acetate copolymer and a polyolefin resin can give a film unsuitable for use as a substrate film for pressure-sensitive adhesive sheets, when the proportion of the ethylene/vinyl acetate copolymer is lower than 20% by weight. Specifically, this film, when used as a substrate film for a pressure-sensitive adhesive sheet for application to the skin, considerably curls upon removal of the release paper from the pressure-sensitive adhesive sheet and thus impairs the handleability which is required of the pressure-sensitive adhesive sheet for application to the skin when it is applied to the skin. In particular, according to the present invention, the proportion of the ethylene/vinyl acetate copolymer in the resin composition comprising the ethylene/vinyl acetate copolymer and a polyolefin resin is preferably in the range of from 40 to 70% by weight.

The polyolefin resin to be used in the present invention in combination with the ethylene/vinyl acetate copolymer described above is at least one member selected from the group consisting of polyethylene, polypropylene, ethylene/propylene copolymers, and mixtures of these and having a melt flow rate in the range of from 0.3 to 4.0 g/10 min. A preferred polyolefin resin among these according to the present invention is the linear low-density polyethylene known as metallocene polyethylene because it can give a film having considerably reduced susceptibility to blocking. In particular, according to the present invention, preferred metallocene polyethylene is one having a melt flow rate in the range of from 2.0 to 3.0 g/10 min.

According to the present invention, however, polypropylene and reactor TPO which have a melt flow rate in the range of, for example, from 0.3 to 4.0 g/10 min as shown above, preferably from 1.0 to 3.0 g/10 min, can be used as polyolefin resins besides metallocene polyethylene.

Metallocene polyethylene and reactor TPO, which are usable in the present invention, are already well known. Metallocene polyethylene is a linear low-density polyethylene resin obtained by polymerizing ethylene using a metallocene catalyst. Compared to the low-density polyethylene resins obtained with conventional Ziegler-Natta catalysts, metallocene polyethylene has evenness of molecular-weight distribution and compositional distribution and is greatly improved in nontackiness, impact strength, etc. Commercial products including, e.g., "Carnel" manufactured by Japan Polychem Corp. are available.

Furthermore, reactor TPO is one of the olefin-based thermoplastic elastomers (TPO) having a melting point of 100°C or higher, and usually is a block or graft copolymer having polypropylene (in some cases, polybutene) and an ethylene/propylene copolymer component. "Catalloy" manufactured by Montell Corp., "PER" manufactured by Tokuyama Corp., "Newcon" manufactured by Chisso Corp., and the like are known.

As stated above, the resin composition comprising the ethylene/vinyl acetate copolymer and polyolefin resin both described above has excellent calenderability and can be easily formed into a film by calendering. According to need, embossing can be conducted during this calendering to regulate the surface state at will, i.e., to regulate the surface so as to have any of surface states ranging, for example, from a smooth surface, e.g., a satin surface, to a deeply embossed surface, e.g., a silk fabric texture.

The substrate film to be used in the pressure-sensitive adhesive sheet for application to the skin according to the present invention preferably has a modulus at 10% of 5.0 N/19-mm width or lower in order that the film, after application to the skin, might well conform to the skin and be flexible without giving an uncomfortable feeling and that the film, despite the flexibility, might have the strength required of substrate films for pressure-sensitive adhesive sheets for application to the skin. The lower the modulus at 10%, the better the wear feeling after application to the skin. However, since modulus at 10% is generally proportional to breaking strength, it is preferred according to the present invention that the film obtained should have a breaking strength of 20 N/19-mm width or higher so as to have the practical strength required of substrate films for pressure-sensitive adhesive sheets.

Moreover, the thickness of the substrate film to be used in the pressure-sensitive adhesive sheet for application to the skin according to the present invention is generally in the range of from 20 to 200 µm in order that the film, when used as a substrate film for pressure-sensitive adhesive sheets for application to the skin which include first-aid plasters, might give no uncomfortable feeling to the skin, well conform to the skin, and be flexible and suitable for the use as in the case described above.

In addition, the substrate film to be used in the pressure-sensitive adhesive sheet for application to the skin according to the present invention should have excellent surface slip properties in order that the pressure-sensitive adhesive drug sheet for application to the skin obtained using the film might be prevented, after application to the skin, from readily peeling off the skin due to friction with clothes or from suffering edge lifting. For attaining this, the coefficient of dynamic friction thereof as measured under the conditions of a load of 200 g and a test speed of 100 mm/min is preferably 1.5 N or lower, especially preferably in the range of from 0.3 to 1.0 N.

The substrate film to be used in the pressure-sensitive adhesive sheet for application to the skin according to the present invention is excellent, by itself, in adhesion to pressure-sensitive adhesives, i.e., in anchoring effect. It is, however, possible to treat a surface of the substrate film with a corona discharge or coat the surface with any of already known various primers to thereby enhance the anchoring effect of a pressure-sensitive adhesive on the substrate film prior to the formation of a pressure-sensitive adhesive layer on the substrate film. Preferably, however, a corona discharge treatment enables a pressure-sensitive adhesive layer to be formed on the substrate film with a sufficient anchoring force without necessitating a primer treatment of the treated surface.

The pressure-sensitive adhesive sheet for application to the skin according to the present invention can be obtained by forming a pressure-sensitive adhesive layer for application to the skin on the substrate film described above. Any desired pressure-sensitive adhesive can be used as the pressure-sensitive adhesive without particular limitations as long as it has reduced skin-irritating properties and is usable as a medical pressure-sensitive adhesive. Besides the conventionally known acrylic pressure-sensitive adhesives and natural-rubber-based pressure-sensitive adhesives, usable examples include pressure-sensitive adhesives of the synthetic-rubber-based type, silicone type, and vinyl ether type. In the present invention, however, it is preferred to use an acrylic pressure-sensitive adhesive among various pressure-sensitive adhesives from the standpoint of dermatology, for example, because acrylic pressure-sensitive adhesives are less allergenic for the skin.

In particular, it is preferred according to the present invention to use an emulsion type acrylic pressure-sensitive adhesive obtained by emulsion-copolymerizing an alkyl acrylate in which the alkyl moiety has 4 to 12 carbon atoms such as 2-ethylhexyl acrylate or isononyl acrylate, which gives a polymer having a low glass transition point, with a monomer mixture comprising (meth)acrylic acid and an alkyl methacrylate in which the alkyl moiety has 1 to 4 carbon atoms.

However, according to the present invention, the pressure-sensitive adhesive for application to the skin is not limited to the emulsion type, and may be used in any desired form such as, e.g., an organic solvent-based type or hot-melt type. These pressure-sensitive adhesives may be used alone or in combination of two or more thereof.

The thickness of the pressure-sensitive adhesive layer is generally in the range of from 20 to 80 µm, preferably in the range of from 30 to 60 µm, from the standpoint of adhesion to the skin.

For forming a pressure-sensitive adhesive layer on the substrate film, a solution of a pressure-sensitive adhesive may be directly applied to the substrate film and dried. Alternatively, a hot-melt pressure-sensitive adhesive may be melted and directly applied to the substrate film with an extruder. However, for preventing the substrate film from stretching or bending (curling) unnecessarily, it is preferred to use the so-called transfer method, in which a pressure-sensitive adhesive layer is formed beforehand on one side of a release paper by an appropriate technique and the substrate film is laminated to this pressure-sensitive adhesive layer to transfer the pressure-sensitive adhesive layer to the substrate film.

Besides being usable as a dressing, a surgical tape, or the like, the pressure-sensitive adhesive sheet for application to the skin according to the present invention can be used in the form of a first-aid plaster obtained by disposing a liquid-absorbing pad for wound protection, such as a fabric, e.g., a gauze, or a sponge pad, on the surface of the pressure-sensitive adhesive layer (preferably in the central area).

The present invention will be explained below by reference to Examples, but the invention should not be construed as being limited by these Examples in any way. The polyolefin resins shown in the Tables have the following properties. The number of parts of each of the resins used for the resin compositions shown in the following Examples and Tables is the number of parts by weight.

### Ultrathene YX11:

Ethylene/vinyl acetate copolymer manufactured by Tosoh Corp.; vinyl acetate content, 32% by weight; Mw = 1.26 × 10⁵; MFR = 0.25 g/10 min

### Ultrathene 635:

Ethylene/vinyl acetate copolymer manufactured by Tosoh Corp.; vinyl acetate content, 25% by weight; Mw = 6.3 × 10⁴; MFR = 5.7 g/10 min

### Ultrathene 751:

Ethylene/vinyl acetate copolymer manufactured by Tosoh Corp.; vinyl acetate content, 28% by weight; Mw = 5.5 × 10⁴; MFR = 2.4 g/10 min

### Catalloy KS353P:

Reactor TPO manufactured by Montell; MFR = 0.45 g/10 min

### Carnel KF282:

Metallocene polyethylene resin manufactured by Japan Polychem Corp.; MFR = 2.2 g/10 min

### EG7F:

Polypropylene resin manufactured by Japan Polychem Corp.; MFR = 1.3 g/10 min

### EXAMPLE 1

### (Film Production by Calendering and Evaluation of Calenderability)

A resin composition having the makeup shown in Table 1 was formed into a film having a thickness of 100 µm with a two-roll mill having a surface temperature of 170°C. During this processing, the resin composition was examined for calenderability based on releasability and haul-off properties. Namely, with respect to releasability, the case in which the resin composition was easily released from the rolls when the film was peeled from the rolls is indicated by A, while the case in which the resin was difficult to take off the rolls is indicated by B. With respect to haul-off properties, the case in which the film could be hauled off without arousing any trouble when peeled from the rolls is indicated by A, while the case in which the film sagged or broke when peeled from the rolls is indicated by B.

### (Production of Substrate Film and Evaluation of Blocking Resistance)

Subsequently, the resin composition having calenderability, which was evaluated based on the releasability and haul-off properties, was kneaded with a closed type mixing machine and passed through a strainer. Thereafter, a film having a thickness of 70 µm was produced with a four-roll inverted L calender having a roll surface temperature of 180°C, and this film was wound into a roll. This rolled film was then unwound and examined for blocking in this operation. The case in which no blocking was observed is indicated by A, while the case in which blocking was observed is indicated by B.

### (Production of Pressure-Sensitive Adhesive Sheet and Evaluation of Properties Thereof)

Two parts by weight of acrylic acid, 93 parts by weight of 2-ethylhexyl acrylate, and 10 parts by weight of methyl methacrylate were subjected to emulsion copolymerization in a nitrogen atmosphere to obtain an emulsion type acrylic copolymer. This emulsion type acrylic copolymer was applied to the treated side of a release paper which had been treated on one side with a silicone, in such an amount as to result in a thickness on a dry basis of 40 µm. The coating was dried at 120°C for 3 minutes to form a pressure-sensitive adhesive layer on the release paper.

One side of the 70 µm-thick film obtained by calendering was subjected to a corona discharge treatment. The pressure-sensitive adhesive layer on the release paper was laminated to the corona discharge-treated side of the film to produce a pressure-sensitive adhesive sheet having the release paper.

The pressure-sensitive adhesive sheet thus obtained was evaluated for modulus at 10%, wear feeling, coefficient of dynamic friction, surface slip property, susceptibility to curling, and peel strength by the following methods. The results are shown in Table 1. Modulus at 10%

The pressure-sensitive adhesive sheet obtained was cut into a size having a width of 19 mm and a length of 100 mm. The load at 10% elongation as measured in an atmosphere having a temperature of 23±2°C and a relative humidity of 65±15% under the conditions of a chuck-to-chuck distance of 50 mm and a pulling rate of 300 mm/min was taken as the modulus at 10%. When this modulus at 10% is 5.0 N/19-mm width or lower, the pressure-sensitive adhesive sheet is judged to be flexible.

### Wear Feeling

The pressure-sensitive adhesive sheet obtained was cut into a size having a width of 19 mm and a length of 72 mm. This pressure-sensitive adhesive sheet was wound around the second joints of the second finger, third finger, and fourth finger of a hand of a subject so that the adhesive sheet overlapped. These joints were bent and stretched to evaluate the wear feeling in terms of the degree of a feeling of tightness in the bending and stretching. The case in which the feeling of tightness was nil, slight, or strong is indicated by A, B, or C, respectively.

### Coefficient of Dynamic Friction

In an atmosphere having a temperature of 23±2°C and a relative humidity of 65±15%, a slide plate made of stainless steel having a size of 63 mm square and a weight of 200 g was placed on the surface of the substrate film of the pressure-sensitive adhesive sheet obtained. The slide plate was pulled at a pulling rate of 100 mm/min, and the load as measured when the slide plate had began to move and come to have a stable value was taken as the coefficient of dynamic friction. When the coefficient of dynamic friction is 1.5 N or lower, the pressure-sensitive adhesive sheet is judged to be excellent in the surface slip properties of the substrate film.

### Slip Property

The pressure-sensitive adhesive sheet obtained was cut into a size having a width of 19 mm and a length of 72 mm. This pressure-sensitive adhesive sheet was applied to a knee of a subject so that the lengthwise direction for the pressure-sensitive adhesive sheet became horizontal (extended from the inner side to the outer side of the knee). After 24 hours, this pressure-sensitive adhesive sheet was examined for the degree of peeling due to friction with clothes and evaluated. The case in which the pressure-sensitive adhesive sheet had undergone no peeling, undergone slight end lifting, or undergone end peeling is indicated by A, B, or C, respectively.

### Susceptibility to Curling

The pressure-sensitive adhesive sheet obtained was cut into a size having a width of 19 mm and a length of 72 mm. In an atmosphere having a temperature of 23±2°C and a relative humidity of 65±15%, the release paper was removed from the pressure-sensitive adhesive sheet. The susceptibility to curling was evaluated in terms of the degree of the resultant curling. The case in which no curling was observed is indicated by A. The case in which curling was observed but this curling was not practically problematic is indicated by B. The case in which the pressure-sensitive adhesive sheet curled considerably and was difficult to apply to the skin is indicated by C.

### Peel Strength

The pressure-sensitive adhesive sheet obtained was cut into a size having a width of 19 mm and a length of 72 mm, and through-holes having a diameter of 1 mm were formed therein in the arrangement shown in Fig. 1. This pressure-sensitive adhesive sheet was applied to and wound around the second joints of the second finger, third finger, and fourth finger of a hand of a subject so that the adhesive sheet overlapped. After 1 hour, the pressure-sensitive adhesive sheet was peeled from the fingers and the peel strength was evaluated based on how the pressure-sensitive adhesive sheet was stripped off. Namely, the case in which the pressure-sensitive adhesive sheet was stripped off without arousing any trouble is indicated by A. The case in which the pressure-sensitive adhesive sheet was stripped off without breaking although it stretched considerably is indicated by B. The case in which the pressure-sensitive adhesive sheet broke is indicated by C.

### EXAMPLES 2 TO 8

Resin compositions were examined for calenderability in the same manner as in Example 1, except that the resin compositions each shown in Table 1 were used. Thereafter, each resin composition was formed into a film having a thickness of 70 µm by calendering. The films obtained were examined for blocking in the same manner as in Example 1. The results are shown in Table 1.

The films thus obtained were used as substrate films to produce pressure-sensitive adhesive sheets in the same manner as in Example 1. The pressure-sensitive adhesive sheets thus obtained were examined for modulus at 10%, wear feeling, coefficient of dynamic friction, surface slip property, susceptibility to curling, and peel strength in the same manners as in Example 1. The results are shown in Table 1.

### COMPARATIVE EXAMPLES 1 TO 12

Resin compositions were examined for calenderability in the production of 100 µm-thick films through calendering in the same manner as in Example 1, except that resin compositions having the makeups shown in Table 2 and Table 3 were used. The resin compositions having calenderability were formed into a film having a thickness of 70 µm in the same manner. The films obtained were examined for blocking in the same manner as in Example 1. The results are shown in Table 2 and Table 3.

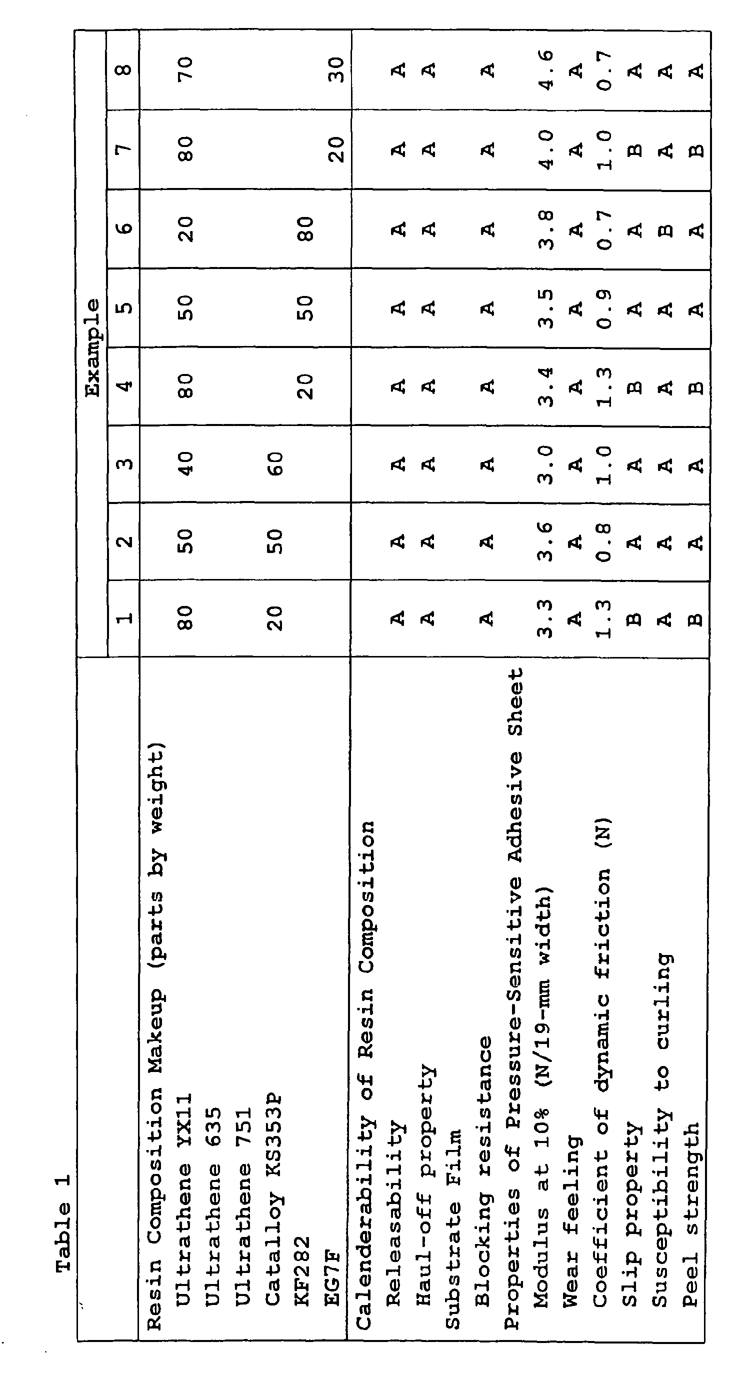

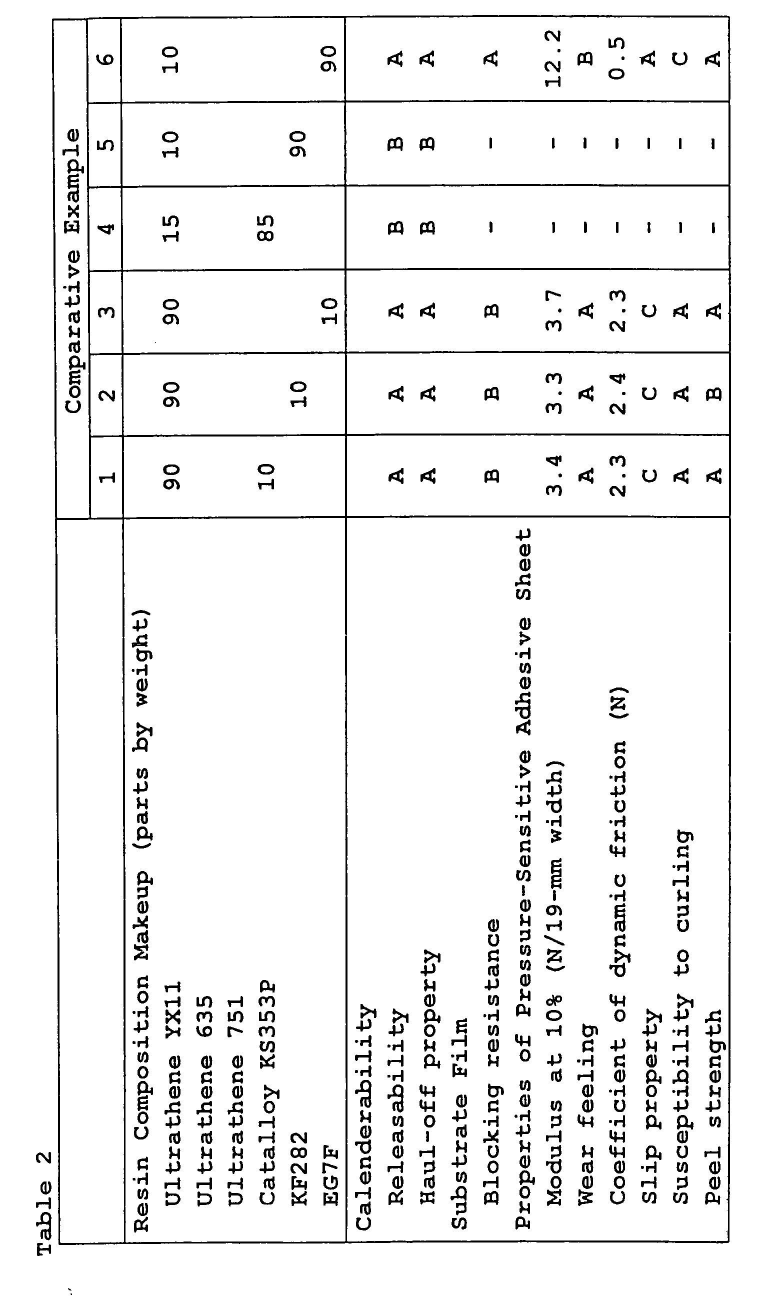

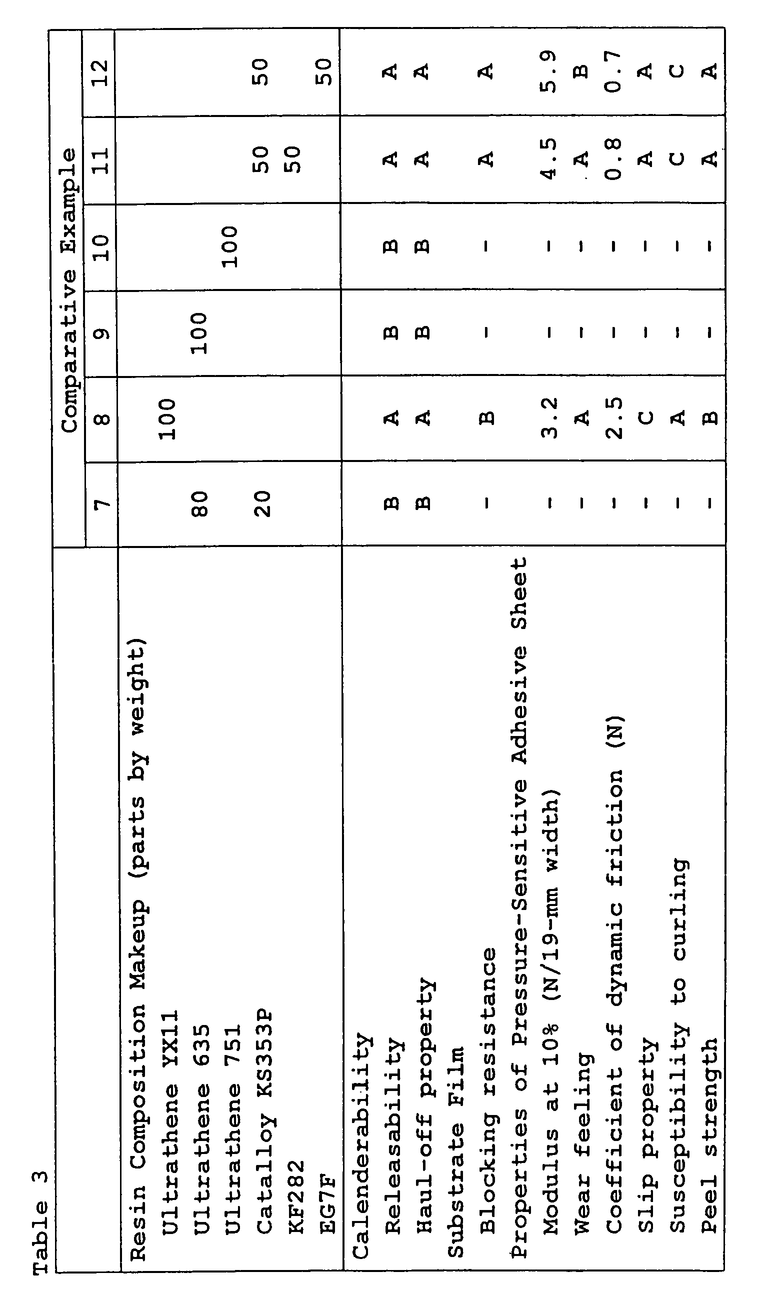

In each of Comparative Examples 1 to 3 and 8, an ethylene/vinyl acetate copolymer specified in the present invention is used but the proportion thereof in the composition with a polyolefin resin is too large. Because of this, these compositions give a film which is highly susceptible to blocking, although they have excellent calenderability. In contrast, in Comparative Examples 4 and 5, the proportion of the metallocene polyethylene resin and that of the reactor TPO to the ethylene/vinyl acetate copolymer specified in the present invention each are too large. As a result, the resin compositions have poor calenderability and a target film cannot be obtained therefrom.

In Comparative Example 6, the proportion of the polypropylene resin to the ethylene/vinyl acetate copolymer specified in the present invention is too large. Because of this, the film obtained is rigid and gives a poor wear feeling on the skin, although excellent in slip properties. In Comparative Examples 9 and 10, the ethylene/vinyl acetate copolymers employed have neither the weight-average molecular weight nor melt flow rate specified in the present invention and are not used in combination with a polyolefin resin. Because of this, a film cannot be obtained therefrom by calendering.

In Comparative Examples 11 and 12, an ethylene/vinyl acetate copolymer specified in the present invention is not used and polyolefin resins specified in the present invention are used as the only polymers and subjected to calendering. Although films can be obtained therefrom, the films obtained have considerable susceptibility to curling.

Comparative Example 7 employs a combination of an ethylene/vinyl acetate copolymer and a polyolefin resin in a proportion specified in the present invention. However, the ethylene/vinyl acetate copolymer has a lower weight-average molecular weight and a higher melt flow rate than the values specified in the present invention. Because of this, the composition shows poor haul-off properties in calendering and a target film cannot be obtained therefrom.

In contrast to those Comparative Examples, the resin compositions according to the present invention have excellent calenderability and give films which are flexible and well conform to the skin. Because of this, the pressure-sensitive adhesive sheets obtained using such films as substrate films, after application to the skin, give an excellent wear feeling without giving an uncomfortable feeling. Furthermore, these adhesive films have excellent film surface slip properties and are not susceptible to curling.

Namely, the pressure-sensitive adhesive sheets for application to the skin according to the present invention showed excellent results in all evaluations. The pressure-sensitive adhesive sheets are flexible, have practically sufficient strength despite the flexibility, and are excellent also in surface slip properties and unsusceptibility to curling. These adhesive sheets have a well-balanced combination of properties required of pressure-sensitive adhesive sheets for application to the skin and are especially suitable for use as pressure-sensitive adhesive sheets for application to the skin which are to be used as first-aid plasters.

The pressure-sensitive adhesive sheets according to Comparative Examples 1 to 3 are ones obtained using substrate films respectively made of resin compositions which consist of an ethylene/vinyl acetate copolymer and a polyolefin resin and in which the proportion of the ethylene/vinyl acetate copolymer is larger than that specified in the present invention. Because of this, as apparent from a comparison with the pressure-sensitive adhesive sheets according to the present invention, the surface (i.e., film side) of each of the pressure-sensitive adhesive sheets of Comparative Examples 1 to 3 has a high coefficient of dynamic friction and poor slip properties. In contrast, the pressure-sensitive adhesive sheets according to Comparative Examples 4 to 6 are ones obtained using substrate films respectively made of resin compositions which consist of an ethylene/vinyl acetate copolymer and a polyolefin resin and in which the proportion of the ethylene/vinyl acetate copolymer is smaller than that specified in the present invention. Because of this, as apparent from a comparison with the pressure-sensitive adhesive sheets according to the present invention, the pressure-sensitive adhesive sheets of Comparative Examples 4 to 6 have considerable susceptibility to curling and are inferior also in wear feeling to the pressure-sensitive adhesive sheets according to the present invention.

The pressure-sensitive adhesive sheet according to Comparative Example 8 is one obtained using as a substrate film a film formed only from an ethylene/vinyl acetate copolymer specified in the present invention. This pressure-sensitive adhesive sheet has poor slip properties. The pressure-sensitive adhesive sheets of Comparative Examples 11 and 12 each are one obtained using a substrate film made of a polyolefin resin alone. These pressure-sensitive adhesive films have considerable susceptibility to curling.

In contrast, the pressure-sensitive adhesive sheets according to the present invention each are flexible and excellent in wear feeling and, despite this, have practically sufficient strength. Furthermore, they have excellent surface slip properties and are free from curling. Consequently, these adhesive sheets can be advantageously used as pressure-sensitive adhesive sheets for application to the skin.

As described above, the pressure-sensitive adhesive sheet for application to the skin according to the present invention employs a substrate film made of a resin composition comprising an ethylene/vinyl acetate copolymer having properties in certain ranges and a polyolefin resin. This pressure-sensitive adhesive sheet, of course, combines flexibility and strength, which are practically necessary. Furthermore, the pressure-sensitive adhesive sheet, after application to the skin, well fits with and conforms to the skin and, hence, gives an excellent wear feeling without giving an uncomfortable feeling. In addition, since the pressure-sensitive adhesive sheet has excellent surface slip properties, it does not readily peel off the skin even upon friction with clothes, etc.

Furthermore, the resin composition, in calendering, is excellent in releasability from the rolls and haul-off properties, i.e., calenderability, and even a thin film can be stably produced therefrom. In addition, when a film thus obtained and wound into a roll is unwound in order to use it as a substrate film for a pressure-sensitive adhesive sheet and to form, e.g., a pressure-sensitive adhesive layer thereon, then the film shows no blocking. Even when the release paper disposed on the pressure-sensitive adhesive layer formed on the substrate film is removed, the substrate film does not curl. Consequently, the pressure-sensitive adhesive sheet for application to the skin according to the present invention can be advantageously used as a pressure-sensitive adhesive sheet for application to the skin, which includes a first-aid plaster.

While the invention has been described in detail and with reference to specific embodiments thereof, it will be apparent to one skilled in the art that various changes and modifications can be made therein without departing from the scope thereof.

This application is based on Japanese patent application No. 2003-179230 filed June 24, 2003, the entire contents thereof being hereby incorporated by reference.

## Claims

1. A pressure-sensitive adhesive sheet for application to the skin, which comprises (1) a substrate film comprising a resin composition comprising from 20 to 80% by weight of an ethylene/vinyl acetate copolymer having a weight-average molecular weight higher than 1×10⁵ and not higher than 1×10⁶, a melt flow rate of 1.0 g/10 min or lower, and a vinyl acetate content higher than 28% by weight and not higher than 35% by weight and from 80 to 20% by weight of at least one polyolefin resin selected from the group consisting of polyethylene, polypropylene, ethylene/propylene copolymers, and mixtures thereof and having a melt flow rate in the range of from 0.3 to 4.0 g/10 min, and (2) a pressure-sensitive adhesive layer for application to the skin.

2. The pressure-sensitive adhesive sheet for application to the skin of claim 1, wherein said substrate film has a modulus at 10% of 5.0 N/19-mm width or lower.

3. The pressure-sensitive adhesive sheet for application to the skin of claim 1, wherein the coefficient of dynamic friction of the substrate film, as measured under the conditions of a load of 200 g and a test speed of 100 mm/min, is 1.5 N or lower.

4. The pressure-sensitive adhesive sheet for application to the skin of claim 1, wherein the thickness of the substrate film is from 20 to 200 µm.

5. The pressure-sensitive adhesive sheet for application to the skin of claim 1, wherein the pressure-sensitive adhesive layer comprises an acrylic pressure-sensitive adhesive.

6. The pressure-sensitive adhesive sheet for application to the skin of claim 5, wherein the acrylic pressure-sensitive adhesive comprises a copolymer obtainable by copolymerizing (a) an alkyl acrylate in which the alkyl moiety has 4 to 12 carbon atoms with (b) a monomer mixture comprising (meth)acrylic acid and an alkyl methacrylate in which the alkyl moiety has 1 to 4 carbon atoms.

7. A first-aid plaster which comprises the pressure-sensitive adhesive sheet for application to the skin of any one of claims 1 to 6 and a liquid-absorbing pad disposed on the surface of the pressure-sensitive adhesive layer.

8. A process for producing the pressure-sensitive adhesive sheet for application to the skin of claim 1, which comprises forming the resin composition into a film by calendering, and forming a pressure-sensitive adhesive layer on the substrate film.

9. The process of claim 8 for producing a pressure-sensitive adhesive sheet for application to the skin, wherein the thickness of the substrate film is from 20 to 200 µm.

10. The process of claim 8 for producing a pressure-sensitive adhesive sheet for application to the skin, wherein the pressure-sensitive adhesive layer comprises an acrylic pressure-sensitive adhesive.

11. The process of claim 10 for producing a pressure-sensitive adhesive sheet for application to the skin, wherein the acrylic pressure-sensitive adhesive comprises a copolymer obtainable by copolymerizing (a) an alkyl acrylate in which the alkyl moiety has 4 to 12 carbon atoms with (b) a monomer mixture comprising (meth)acrylic acid and an alkyl methacrylate in which the alkyl moiety has 1 to 4 carbon atoms.
